# EUROPEAN PATENT APPLICATION

(11) **EP 3 785 629 A1**
(43) Date of publication of application: **03.03.2021**
(21) Application number: 19792057.2
(22) Date of filing: 15.04.2019
(51) Int. Cl.: A61B 5/11, A61H 1/02

(54) **WALKING MANNER DISPLAY METHOD, WALKING MANNER DISPLAY SYSTEM AND WALKING MANNER ANALYSIS DEVICE**

(30) Priority: 26.04.2018 JP 2018084952
(71) Applicant: Hitachi High-Tech Corporation, Minato-ku Tokyo 105-6409 (JP)
(72) Inventor: FUKUI Daisuke, Tokyo 100-8280 (JP); EGI Masashi, Tokyo 100-8280 (JP); NAKAGAWA Hiromitsu, Tokyo 100-8280 (JP); TANAKA Takeshi, Tokyo 100-8280 (JP); MIYAKE Masatoshi, Tokyo 105-8717 (JP); ONO Takashi, Tokyo 105-8717 (JP); HORIKOSHI Nobuya, Tokyo 105-8717 (JP); NOGUCHI Minori, Tokyo 105-8717 (JP)
(74) Representative: Strehl Schübel-Hopf & Partner
(86) International application number: PCT/JP2019/016215
(87) International publication number: WO 2019/208312

(57) **Abstract**

To provide a walking mode display method, a walking mode display system and a walking mode analyzer each of which allows for analysis of a walking mode of a user and display thereof in an easily understandable manner. The walking mode display method includes: selecting measurement of a walker and measurement of a reference walker to be compared with the walker; displaying a first walking model 604 that displays a walking for one walking step of the walker as an animation; displaying a second walking model 603 that displays a walking for one walking step of the reference walker as the animation; and displaying a magnitude of predetermined feature amount data related to the measurement of the walker and the magnitude of predetermined feature amount data related to the measurement of the reference walker in a comparable manner (607).

## Description

### Technical Field

The present invention relates to a walking mode display method, a walking mode display system and a walking mode analyzer.

### Background Art

In order for a person to move his or her body freely, locomotor organs including bones, joints, muscles and nerves need to function normally. Locomotive syndrome ("locomo") refers to a condition in which one or a plurality of locomotor organs are impaired and movement functions such as standing, walking, running, and sitting are deteriorated. When the movement function deterioration progresses, a trouble occurs even in a daily life. It is said that many locomotor disorders that require a hospital treatment occur after an age of 50, and the locomotor disorders in an elderly lead to a risk of needing support or care. Since locomotor disorders gradually progress, it is recognized that there is a need in prevention, early detection, and appropriate measures of the locomo.

PTL 1 discloses a walking state display system capable of grasping a walking state of a walker. In this system, a subject walks with a walking motion measuring device attached, and an index indicating the walking state of the subject is obtained and displayed. Examples of the index include a stride, a walking cycle, a variation state of a center of gravity, a foot height, a knee height, or the like showing the walking state.

### Citation List

### Patent Literature

PTL 1: JP-A-2012-65723

### Summary of Invention

### Technical Problem

The deterioration in the movement functions of a person is represented as a walking disorder. Thus, it is considered effective to grasp a walking state of a person on which the locomo is detected early and an improvement is promoted, and to inform the subject in an easily understandable manner. PTL 1 discloses that the obtained index is used as a walking motion pattern, and a current walking motion pattern of the subject and an optimal walking motion pattern defined in advance are displayed for comparison, or the index is displayed as a time-series graph, but does not disclose how to display the index specifically.

An object of the invention is to allow for analysis of a walking mode of a walker and display thereof in an easily understandable manner, and further to allow for a presentation of an improvement method, which leads to prevention, early detection, and appropriate measures of locomo.

### Solution to Problem

A walking mode display method that displays a walking mode of a walker, which is an embodiment of the invention, includes: previously storing, for each measurement about walking of a plurality of measurement subjects, skeleton data showing a trajectory of a measurement point of the measurement subject in a three-dimensional space, pitch data defining one walking of the measurement subject in the skeleton data, and feature amount data calculated based on the skeleton data for one walking step of the measurement subject; selecting measurement of the walker and a reference walker to be compared with the walker among the plurality of measurement subjects, the measurement being to be displayed on a display screen; displaying a first walking model that displays, as an animation, walking for one walking step of the walker based on the skeleton data and the pitch data related to the measurement of the walker; displaying a second walking model that displays, as an animation, walking for one walking step of the reference walker based on the skeleton data and the pitch data related to the measurement of the reference walker; and displaying a magnitude of predetermined feature amount data related to the measurement of the walker and a magnitude of predetermined feature amount data related to the measurement of the reference walker in a comparable manner.

Other problems and novel characteristics will become apparent from a description of the description and the accompanying drawings.

### Advantageous Effect

The invention allows for analysis of a walking mode of a walker and display thereof in an easily understandable manner.

### Brief Description of Drawings

[FIG. 1] FIG. 1 is a hardware configuration of a walking mode display system.
[FIG. 2] FIG. 2 is a software configuration of the walking mode display system.
[FIG. 3] FIG. 3 is a diagram showing a flow of walking measurement and creation and updating of a walking mode analysis model.
[FIG. 4] FIG. 4 is a diagram showing a registration flow of domain knowledge.
[FIG. 5] FIG. 5 is a flow of displaying a walking mode of a user.
[FIG. 6A] FIG. 6A is an example of a display screen of the walking mode.
[FIG. 6B] FIG. 6B is an example of the display screen of the walking mode.
[FIG. 7] FIG. 7 is an overall flow of displaying the display screen.
[FIG. 8] FIG. 8 is a display flow of a walking model.
[FIG. 9] FIG. 9 is a display flow of a feature amount.
[FIG. 10] FIG. 10 is a display flow of a walking mode degree.
[FIG. 11] FIG. 11 is a display flow of the domain knowledge.
[FIG. 12] FIG. 12 is data structure of domain knowledge data.
[FIG. 13] FIG. 13 is a data structure of measurement metadata.
[FIG. 14] FIG. 14 is a data structure of skeleton data.
[FIG. 15] FIG. 15 is a data structure of pitch data.
[FIG. 16A] FIG. 16A is a data structure of feature amount data.
[FIG. 16B] FIG. 16B is an example of the feature amount.
[FIG. 17] FIG. 17 is a data structure of feature amount explanation data.
[FIG. 18] FIG. 18 is a data structure of an analysis model.

### Description of Embodiments

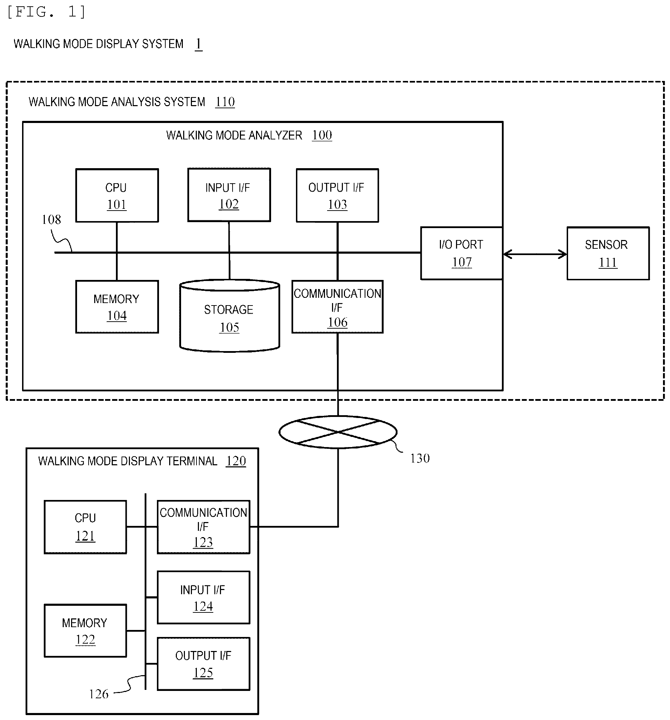

FIG. 1 shows a hardware configuration of a walking mode display system 1. The walking mode display system 1 includes a walking mode analysis system 110 that creates a walking mode analysis model based on walking measurement of a measurement subject and a measurement result thereof, a walking mode display terminal 120 that displays a walking mode of a user analyzed by a walking mode analyzer 100, and a walking mode analyzer 100 of the walking mode analysis system 110 and the walking mode display terminal 120 are connected by a network 130.

The walking mode analyzer 100 includes a central processing unit (CPU) 101, an input interface (I/F) 102, an output I/F 103, a memory 104, a storage 105, a communication I/F 106, and an I/O port 107, which are connected by an internal bus 108 and can be implemented as a general-purpose computer. The input I/F 102 is connected to an input device such as a keyboard or a mouse, and the output I/F 103 is connected to a display or a printer to implement a graphical user interface (GUI) for an operator. The communication I/F 106 is an interface for connecting to the network 130. The storage 105 is generally configured with a non-volatile memory such as an HDD, a ROM, and a flash memory, and stores a program to be executed by the walking mode display system 1, data to be processed by the program, or the like. The memory 104 is configured with a random access memory (RAM), and temporarily stores the program, the data required for executing the program, or the like in response to an instruction of the CPU 101. The CPU 101 executes a program loaded from the storage 105 to the memory 104.

The walking mode analyzer 100 is connected to a sensor 111 via the I/O port 107, and forms the walking mode analysis system 110. The walking mode analyzer 100 issues a collection command of sensing data to the sensor 111, the sensor 111 senses walking of the measurement subject in response to the command, and transmits the measurement result to the walking mode analyzer 100. In the present embodiment, an example is described in which a distance sensor of time of flight (TOF) type is used as the sensor 111. The sensor 111 measures a movement (a trajectory) in a three-dimensional space of a measurement point (a joint or the like) of a body of the measurement subject during the walking in order to capture the walking mode of the measurement subject. The distance sensor has an advantage that coordinates of the measurement point in the three-dimensional space can be directly obtained. The sensor 111 is not limited to the distance sensor. For example, the sensor 111 may be a video camera, and image analysis may be performed on a video in which the measurement subject in walking is imaged with the video camera, or a sensor such as an acceleration sensor, an angle sensor, or a gyro sensor may be used. It is also possible to use a plurality of types of sensors. The sensor 111 itself may output the trajectory of the measurement point in the three-dimensional space, and the walking mode analyzer 100 may be configured to calculate the trajectory of the measurement point in the three-dimensional space based on the measurement result or an imaging result of the sensor 111.

The walking mode display terminal 120 displays the walking mode of a user analyzed by the walking mode analyzer 100. The walking mode display terminal 120 includes a CPU 121, a memory 122, a communication I/F 123, an input I/F 124, and an output I/F 125, which are connected by an internal bus 126 and can be implemented as a tablet or a smartphone. Each component of the walking mode display terminal 120 is equivalent to a corresponding component of the walking mode analyzer 100.

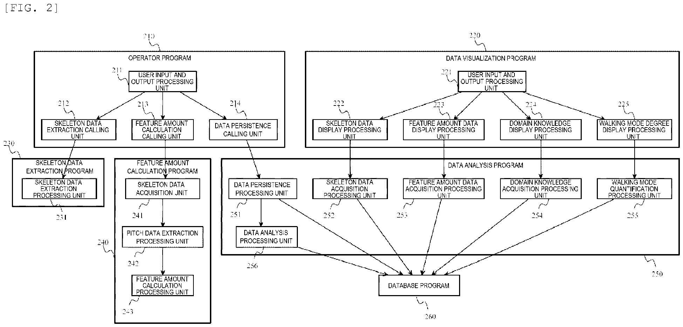

FIG. 2 is a software configuration of the walking mode display system 1, and shows programs executed in the system 1 and relationships thereof. An operator program 210 has a function of measuring the walking and implementing the GUI for creating and updating the walking mode analysis model based on the measurement result thereof. A data visualization program 220 has a function of displaying the walking mode of the user in an easily understandable manner. A skeleton data extraction program 230 is activated by a skeleton data extraction calling unit 212 of the operator program 210 and has a function of extracting skeleton data from the sensing data of the sensor 111. A feature amount calculation program 240 is activated by a feature amount calculation calling unit 213 of the operator program 210 and has a function of calculating a feature amount for analyzing the walking mode of the measurement subject based on the skeleton data. A data analysis program 250 has a function of creating and updating the walking mode analysis model that analyzes the walking mode of the user based on the feature amount, a function of storing measurement data or an analysis model in a database, and a function of acquiring data required for displaying the walking mode or evaluating the walking mode of the user based on the walking mode analysis model when analysis data is displayed by the data visualization program 220. A database program 260 has a function of storing the measurement data or the analysis model required for the walking mode display system 1 into the storage 105 and managing the measurement data or the analysis model. The operator program 210, the skeleton data extraction program 230, the feature amount calculation program 240, the data analysis program 250, and the database program 260 are programs executed by the walking mode analyzer 100, and the data visualization program 220 is a program executed by the walking mode display terminal 120.

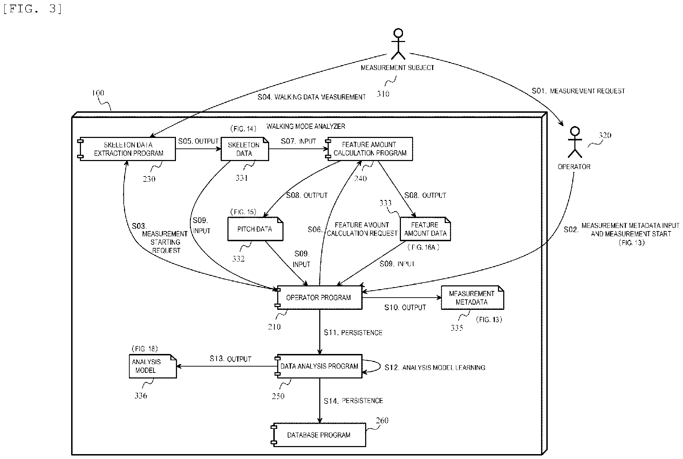

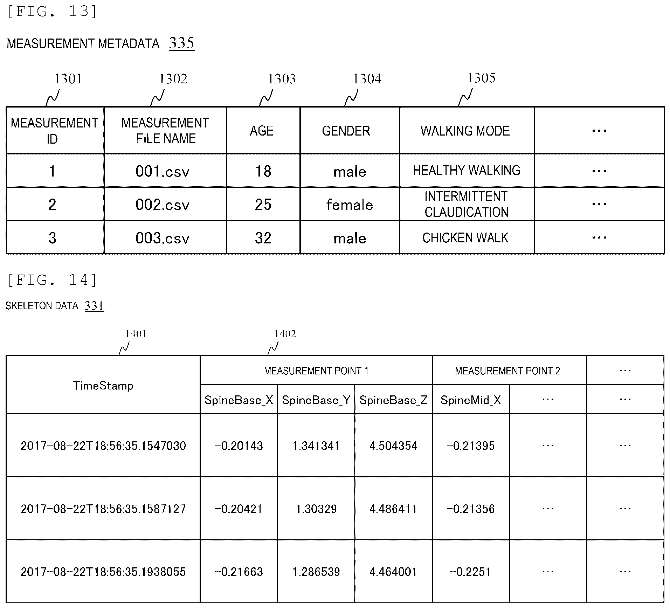

A flow of the walking mode analyzer 100 performing walking measurement and creating and updating of the walking mode analysis model based on the measurement result thereof is described with reference to FIG. 3. A measurement subject 310 requests an operator 320 to perform measurement (S01), and the operator 320 inputs measurement metadata of the measurement subject 310 into the system and instructs a user input and output processing unit 211 of the operator program 210 so as to start the measurement (S02). A data structure of the measurement metadata is shown in FIG. 13.

Measurement metadata 335 is created for each measurement (S10). The measurement metadata includes a measurement ID 1301 for uniquely identifying the data, a measurement file name 1302 for storing the measurement result of the sensor 111, and basic data 1303 to 1305 related to the measurement subject 310. The basic data is used as a determination of a population of learning data when the walking mode analysis model is learned, or is used as a part of variables of the walking mode analysis model. Examples of the basic data include items such as an age 1303, a gender 1304, and a walking mode 1305.

The skeleton data extraction calling unit 212 (the operator program 210) activates the skeleton data extraction program 230, and the measurement is started (S03) . A skeleton data extraction processing unit 231 measures the walking of the measurement subject (S04) and outputs skeleton data 331 (S05). In the present embodiment, it is assumed that the skeleton data extraction program 230 acquires the sensing data from the sensor 111 online, but it is also possible to input the sensing data acquired by the sensor 111 to a device offline . A data structure of the skeleton data 331 is shown in FIG. 14.

The skeleton data 331 is the trajectory of the measurement point of the measurement subject in the three-dimensional space, and a vertical axis stores time (a time stamp) 1401 and a horizontal axis stores (X, Y, Z) coordinates 1402 of each measurement point. The joint or the like that influences the walking mode is set at the measurement point. The skeleton data 331 is used to extract the feature amount related to the walking of the measurement subject, as to be described later. For this reason, in order to eliminate a difference depending on a physique of each measurement subject, it is desirable to store, for example, the measurement points in the body with less shaking during walking as relative coordinates that are always relative to each other as an origin during walking.

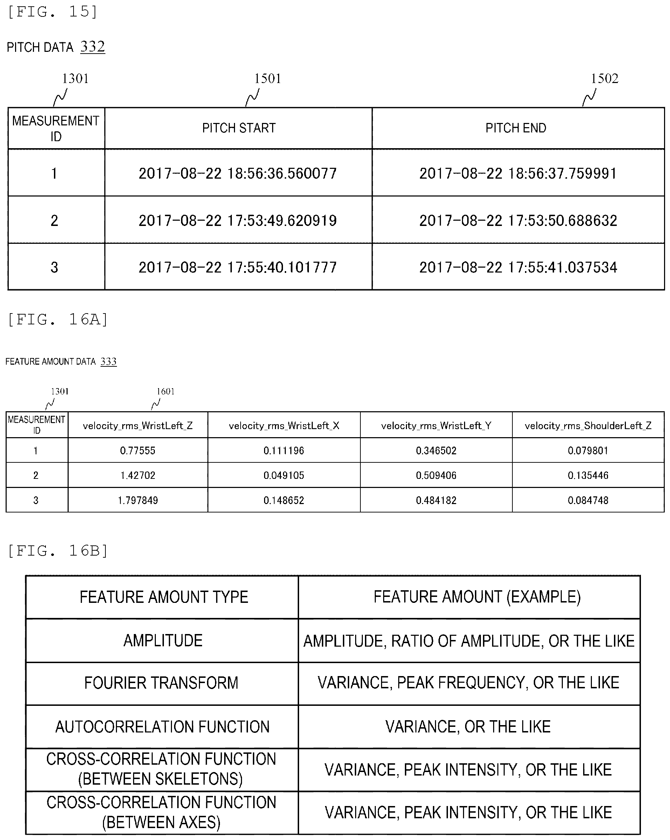

When the skeleton data extraction program 230 extracts the skeleton data 331, a fact that the measurement is ended is returned to the operator program 210. In response to the return, the feature amount calculation calling unit 213 (the operator program 210) activates the feature amount calculation program 240 (S06). First, a skeleton data acquisition unit 241 acquires the skeleton data 331 (S07) . Next, a pitch data extraction processing unit 242 extracts pitch data from the skeleton data 331 (S08). A data structure of pitch data 332 is shown in FIG. 15.

The pitch data 332 defines one walking of the measurement subject in the skeleton data 331. In an example of FIG. 15, the skeleton data designated by the measurement ID 1301 means that the one walking is from time defined by pitch start 1501 to time defined by pitch end 1502. The one walking can be defined, for example, as walking when the walker puts a heel of a right foot on a ground during walking to when the heel of the right foot leaves the ground again, "time when the heel of the right foot lands" and "time when the heel of the right foot leaves the ground" are detected from the sensing data, and are respectively registered in the pitch data 332 as pitch start time and pitch end time.

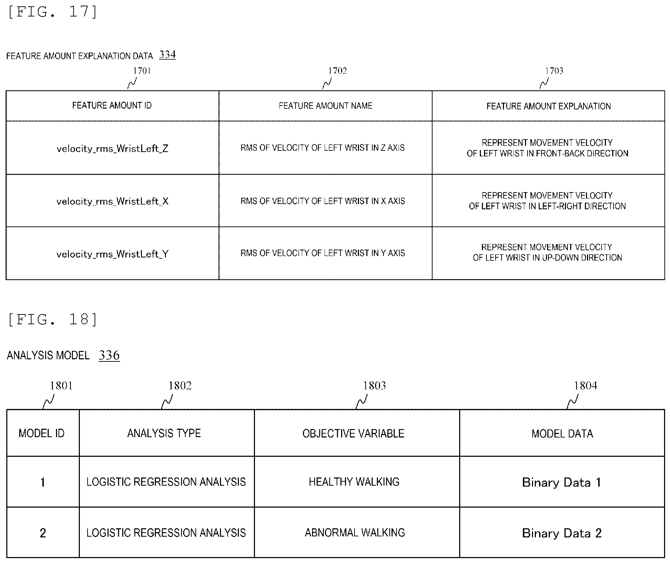

Then, a feature amount calculation processing unit 243 calculates feature amount data based on the skeleton data 331 for one walking step (S08). The calculated feature amount is predetermined in the system as shown in FIG. 16B. The feature amount is the movement and correlation of the joints or axes of the measurement subject during walking, and is calculated based on the skeleton data 331. A data structure of the feature amount data 333 is shown in FIG. 16A. Calculated feature amount 1601 is stored for each measurement ID 1301. When the walking mode measured as to be described later is displayed, the walking mode is displayed such that a content of the feature amount can also be understood by the measurement subject. Therefore, in addition to defining the feature amount calculated by the system, feature amount explanation data 334 is stored in order to explain the content of the feature amount to the user. A data structure of the feature amount explanation data 334 is shown in FIG. 17. The feature amount explanation data 334 includes a feature amount ID 1701 that uniquely identifies the feature amount, a feature amount name 1702 that is a name of the feature amount identified by the feature amount ID, and a feature amount explanation 1703 that explains the content of the feature amount.

A data persistence calling unit 214 of the operator program 210 request the data analysis program 250 to store the measurement metadata 335, the skeleton data 331, the pitch data 332, and the feature amount data 333 obtained by one measurement into the database (S11).

A data analysis processing unit 256 of the data analysis program 250 uses walking data of the measurement subject accumulated in the database as the learning data to learn the walking mode analysis model (S12), and creates or updates an analysis model 336 (S13). A data structure of the analysis model 336 is shown in FIG. 18. The analysis model 336 includes a model ID 1801 that uniquely identifies a model, a model analysis type 1802, an objective variable 1803, and model data 1804. For example, the walking mode analysis model of a model ID "1" in this example is a logistic regression analysis model that calculates a degree (referred to as a "walking mode degree") corresponding to a walking mode "healthy walking", and means that a determination program thereof is "Binary Data 1". Here, a supervised learning model is used as the analysis model, but the analysis model is not limited to a specific model.

A data persistence processing unit 251 of the data analysis program 250 requests the database program 260 to store and manage the measurement metadata 335, the skeleton data 331, the pitch data 332, the feature amount data 333 requested by the operator program 210, and the analysis model 336 created or updated by the data analysis processing unit 256 (S14), and the database program 260 performs the storage and management.

As described above, the walking mode analyzer 100 includes the analysis model 336, and analyzes the walking mode of the user of the system by using the analysis model 336. In the system of the present embodiment, the analyzed walking mode is displayed in an easily understandable manner, or advice for improving the walking mode is given.

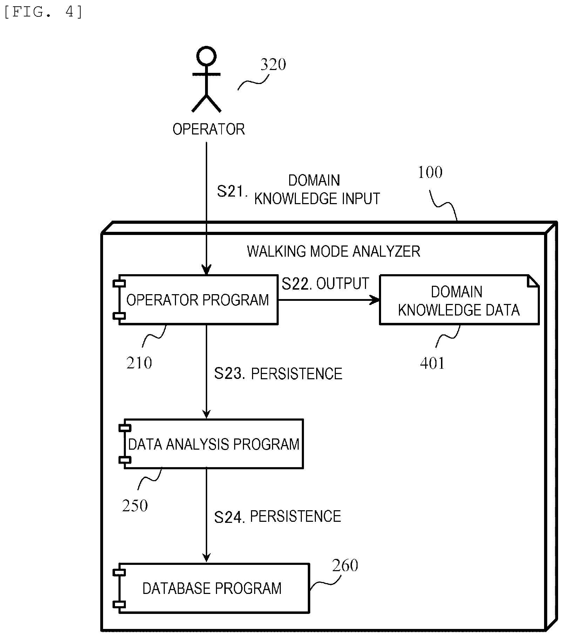

A flow for registering domain knowledge in the system in order to give the advice to the walking mode of the user is described with reference to FIG. 4. The registration flow to the database is similar to the flow of creating and updating the walking mode analysis model, and therefore a detailed description is omitted. A registration flow of the feature amount explanation data 334 described above is also registered in the system in the same flow as in FIG. 4.

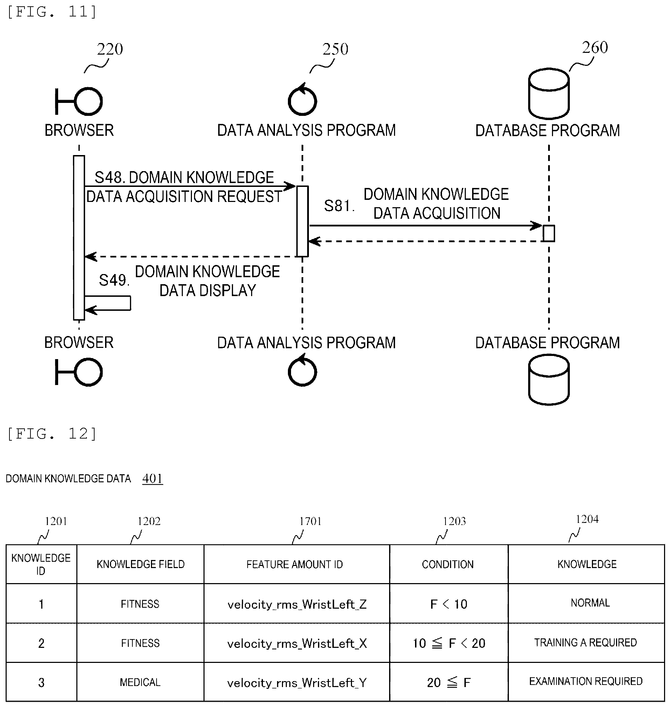

The domain knowledge is specialized knowledge related to a feature amount that characterizes the walking mode of the user. A data structure of domain knowledge data 401 is shown in FIG. 12. The domain knowledge data 401 includes a knowledge ID 1201 that uniquely identifies the domain knowledge, a knowledge field 1202, the feature amount ID 1701, a condition 1203, and knowledge 1204. The knowledge field 1202 indicates which specialized field the domain knowledge is related to. The feature amount ID 1701 shows that the domain knowledge is the specialized knowledge of which feature amount, and specialized knowledge 1204 in a case where the feature amount is a condition shown in the condition 1203 is registered. For example, in a knowledge ID "2", it is registered that the domain knowledge is knowledge of a fitness field and it is recommended to receive training A when a movement velocity of a left wrist in a left-and-right direction is 10 or more and less than 20. In a knowledge ID "3", it is registered that the domain knowledge is knowledge of a medical field and it is recommended to receive an examination when the movement velocity of the left wrist in an up-and-down direction is 20 or more. Contents of the knowledge 1204 are cautions, warnings, advices or the like that are desired to be transmitted to the user when the feature amount data is in a predetermined condition, and the contents thereof are not particularly limited.

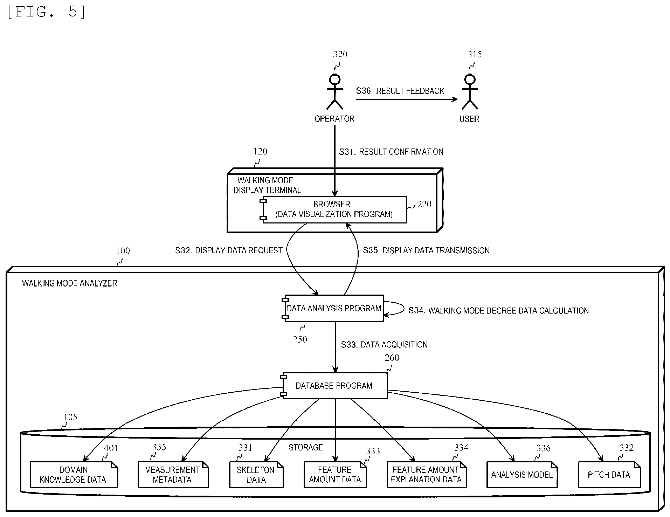

A flow of sensing the walking of the user, analyzing the walking mode of the user by the walking mode analyzer 100, and displaying the walking mode of the user is described with reference to FIG. 5. First, the walking of a user 315 is measured by the walking mode analysis system 110. Similarly to the flow described with reference to FIG. 3, the feature amount data 333 regarding the walking of the user 315 is calculated. In this case, since the walking mode of the user 315 is unknown, there is no walking mode information in the measurement metadata 335. FIG. 5 shows a flow of displaying the walking mode of the user after the feature amount data of the user 315 is calculated and registered in the database. This example shows an example in which the operator 320 operates the walking mode display terminal 120, but the user 315 may directly operate the walking mode display terminal 120.

The operator 320 activates the data visualization program 220 to cause the walking mode display terminal 120 to display the walking mode of the user 315 on a display device (S31) . A user input and output processing unit 221 (see FIG. 2) executes an input of an instruction regarding the activation of the program or contents to be displayed, and a display on a terminal (a display). The data visualization program 220 requests display data from the walking mode analyzer 100 in order to display the walking mode of the user (S32). In response to the request for the display data, the data analysis program 250 acquires data required for visualization via the database program 260 (S33). The required data is stored in the database. That is, the required data includes the measurement metadata 335, the skeleton data 331, the feature amount data 333, the pitch data 332 related to the user 315, the analysis model 336 for analyzing a walking mode, the feature amount explanation data 334, and the domain knowledge data 401. Then, the data analysis program 250 executes processing required to display the acquired data and calculation of walking mode degree data using the analysis model (S34) . The data analysis program 250 transmits the data required for these displays to the walking mode display terminal 120 (S35) . The walking mode display terminal 120 displays the walking mode of the user 315 using the received display data. The operator 320 feeds a result thereof back to the user 315 (S36).

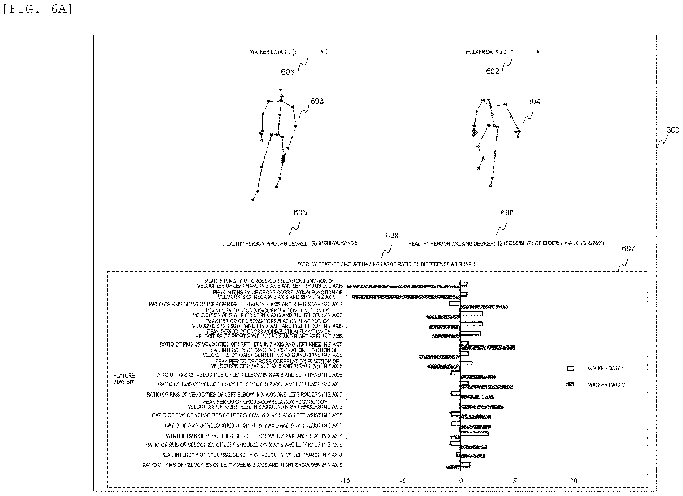

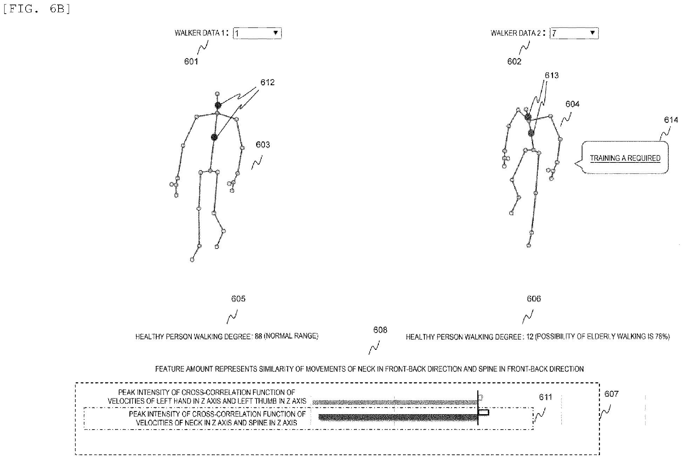

FIGS. 6A and 6B are examples of a display screen of the walking mode of the user displayed by the walking mode display terminal 120. A display screen 600 is adapted to display walking modes of two walkers. This is because it is easy for the user to understand a problem of his or her own walking mode by making it possible to compare a plurality of walking modes at a glance. A walker to be compared (referred to as a reference walker) may be another person having a different walking mode, or may be his or her own past walking mode.

A configuration of the display screen 600 will be described. It is assumed that walker data 1 relates to the reference walker and walker data 2 relates to the user. By selecting the measurement ID of the reference walker and the measurement ID of the user in a first selection column 601 and a second selection column 602, respectively, the walking modes of the reference walker and the user are displayed.

Walking models 603 and 604 display walking of the walker as an animation. A movement of one walking in a skeleton model showing the walker is repeatedly displayed. In the skeleton model of this example, measurement points are circled in the skeleton model that imitates the body of the walker. The walking model 603 and the walking model 604 represent the walking of the reference walker and the user, respectively.

Walking mode degrees 605 and 606 display walking mode degrees obtained by analyzing the walking of the walker using the walking mode analysis model. The walking mode degree 605 is the walking mode degree of the reference walker. This value can be obtained by analysis using a model ID1 in the analysis model shown in FIG. 18. The walking mode degree 606 is the walking mode degree of the user. Similarly, in addition to the analysis based on the model ID1, the walking mode degree 606 can be displayed by analysis using the analysis model for analyzing walking of the elderly.

At a bottom of the display screen 600, a feature amount display column 607 and a feature amount explanation column 608 are provided. The feature amount display column 607 displays the feature amount that characterizes the walking mode of the user among the calculated feature amounts. In this example, the feature amounts related to the walking modes of the reference walker and the user are represented by a bar graph such that the feature amounts are easy to be compared. In the feature amount explanation column 608, explanation of contents displayed in the feature amount display column 607 is displayed. In a case of FIG. 6A, as the feature amount that characterizes the walking mode of the user, a feature amount having a large ratio of difference between the reference walker and the user is shown, and therefore the description thereof is displayed. For example, a predetermined number is selected in order from the feature amount having a large ratio of difference. In addition, the operator can select a specific feature amount by clicking or touching the name or a graph of the feature amount displayed in the feature amount display column 607.

FIG. 6B shows a display screen when the operator selects a feature amount "peak intensity of cross-correlation function of velocities of a neck in a Z axis and a spine in Z axis" on a second line in the screen display of FIG. 6A. At this time, the display changes as follows.
(1) In the feature amount display column 607, a selected feature amount 611 is displayed so as to be visually distinguishable from other feature amounts.
(2) Feature amount explanation of the selected feature amount 611 is displayed in the feature amount explanation column 608.
(3) Measurement points 612 and 613 used for calculating the selected feature amount 611 are distinguishably displayed on the walking models 603 and 604 respectively.
(4) When the domain knowledge exists for the selected feature amount 611, corresponding domain knowledge 614 is displayed.

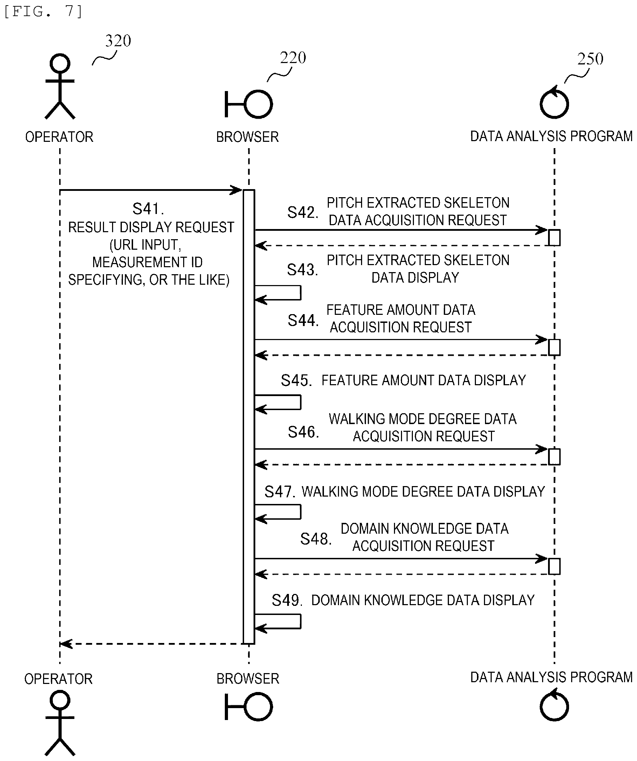

A flow for displaying the display screen 600 is described with reference to FIGS. 7 to 11. FIG. 7 is an overall flow of displaying the display screen. The operator 320 issues a result display request to a browser (the data visualization program) 220 (S41) . The result display request includes a URL of the terminal 120, selection of the measurement IDs of the reference walker and the user that are to be displayed on the display screen, or the like. A pitch extracted skeleton data acquisition request (S42) relates to the display of the walking models 603 and 604. A feature amount data acquisition request (S44) relates to the display of the feature amount display column 607 and the feature amount explanation column 608. A walking mode degree data acquisition request (S46) relates to the display of the walking mode degrees 605 and 606. A domain knowledge data acquisition request (S48) relates to the display of the domain knowledge 614 related to the selected feature amount 611.

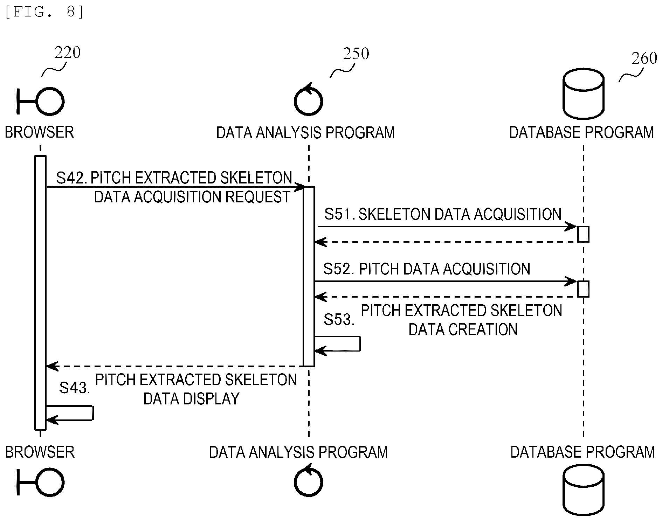

A display flow of the walking model on the display screen 600 is described with reference to FIG. 8. A skeleton data display processing unit 222 (the data visualization program 220, see FIG. 2) issues the pitch extracted skeleton data acquisition request to a skeleton data acquisition processing unit 252 (the data analysis program 250) (S42) . The skeleton data acquisition processing unit 252 acquires the skeleton data (see FIG. 14) and the pitch data (see FIG. 15) related to the selected measurement ID via the database program 260 (S51, S52) . The skeleton data (pitch extracted skeleton data) for the one walking step is extracted from the acquired skeleton data and pitch data (S53). The pitch extracted skeleton data is transmitted from the skeleton data acquisition processing unit 252 to the skeleton data display processing unit 222, and the skeleton data display processing unit 222 animates and displays the walking as the walking model based on the data (S43).

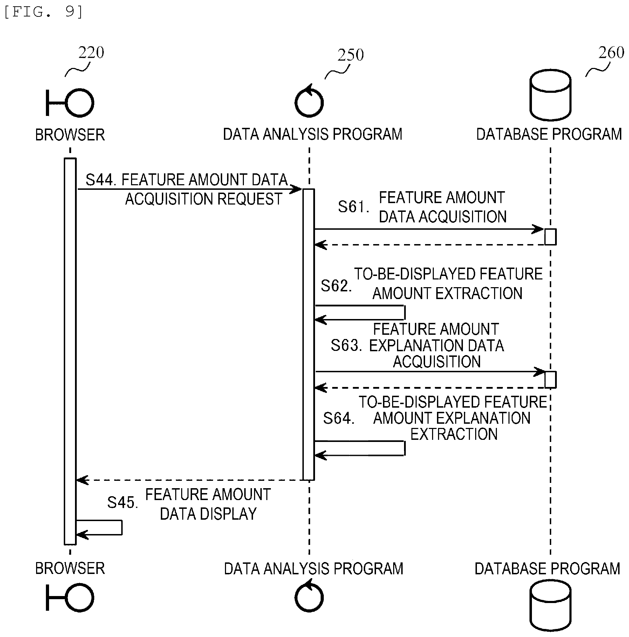

A display flow of the feature amount on the display screen 600 is described with reference to FIG. 9. A feature amount data display processing unit 223 issues the feature amount data acquisition request to a feature amount data acquisition processing unit 253 (S44). The feature amount data acquisition processing unit 253 acquires the feature amount data (see FIG. 16A) related to the selected measurement ID via the database program 260 (S61). The feature amount has an enormous variety since it is intended to accurately analyze the walking mode of the walker. Therefore, it is necessary to select and display the feature amount that is closely related to a feature of the walking mode of the walker. In the example of FIG. 6A, the feature amount to be displayed is selected based on a magnitude of a ratio of a difference between two selected measurement IDs. In this way, the feature amount data acquisition processing unit 253 extracts the feature amount to be displayed based on a predetermined standard (S62). Further, the feature amount data acquisition processing unit 253 acquires the feature amount explanation data (see FIG. 17) via the database program 260 (S63), and extracts feature amount data explanation corresponding to the extracted feature amount data to be displayed (S64). The feature amount data and the feature amount explanation data extracted as the display targets are transmitted from the feature amount data acquisition processing unit 253 to the feature amount data display processing unit 223, and the feature amount data display processing unit 223 creates and displays the bar graph or the like based on the data (S45).

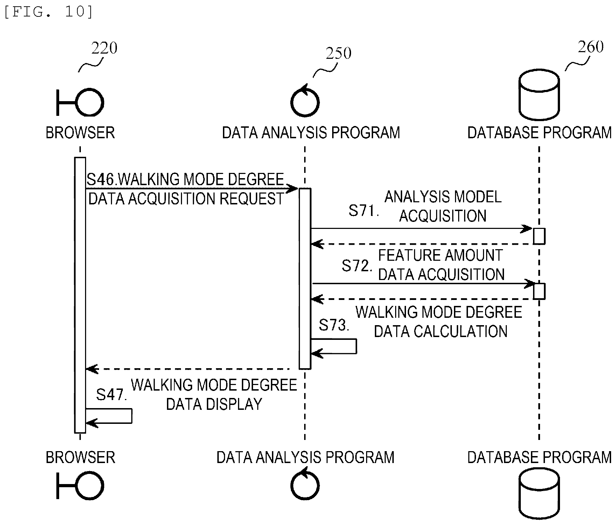

A display flow of the walking mode degree on the display screen 600 is described with reference to FIG. 10. A walking mode degree display processing unit 225 issues the walking mode degree data acquisition request to a walking mode quantification processing unit 255 (S46). The walking mode quantification processing unit 255 acquires the walking mode analysis model (see FIG. 18) and the feature amount data related to the selected measurement ID via the database program 260 (S71). The acquired feature amount data is applied to each analysis model, and walking mode degree data based on each analysis model is calculated (S73). The walking mode degree data is transmitted from the walking mode quantification processing unit 255 to the walking mode degree display processing unit 225, and the walking mode degree display processing unit 225 displays the walking mode degree data based on the data (S47) . A display method is not particularly limited, but when the walking mode is not the healthy walking and some abnormality is observed, a walking mode (in the case of FIG. 6A, "the walking of the elderly") which is an objective variable of the analysis model in which a high value is calculated for the walking mode degree as shown in FIG. 6A is desirable to be displayed as well. For example, the acquired feature amount data is applied to all the analysis models stored in the analysis model 336, and it is sufficient to display the walking mode degree having a largest value at this time.

A display flow of the domain knowledge on the display screen 600 is described with reference to FIG. 11. A domain knowledge display processing unit 224 issues the domain knowledge data acquisition request to a domain knowledge acquisition processing unit 254 (S48). The domain knowledge acquisition processing unit 254 acquires the domain knowledge data (see FIG. 12) via the database program 260 (S81). The acquired domain knowledge data is transmitted from the domain knowledge acquisition processing unit 254 to the domain knowledge display processing unit 224, and the domain knowledge display processing unit 224 displays the domain knowledge based on the data (S49) . As the domain knowledge transmitted by the domain knowledge acquisition processing unit 254, all the domain knowledge stored in the walking mode analyzer 100 may be transmitted, or the domain knowledge related to the feature amount to be displayed may be extracted and transmitted.

While the invention made by the present inventor has been specifically described based on the embodiment, the invention is not limited thereto, and various changes and modifications may be made without departing from the scope of the invention. For example, as the feature amount displayed on the display screen 600, a feature amount that is highly weighted may be selected and displayed in the analysis model in which the high value is calculated for the walking mode degree. In that case, the operator 320 may be able to interactively input the instruction to the browser 220 and switch the feature amounts to be displayed. For example, selection columns of the feature amount are provided on the display screen 600, the "feature amount having a large ratio of difference with the reference walker", the "feature amount having a large contribution to a walking mode degree analysis", or the like are selectable as options, and thus the user 315 can recognize his or her walking mode from a plurality of viewpoints.

### Reference Sign List

- 1: walking mode display system
- 100: walking mode analyzer
- 101: CPU
- 102: input I/F
- 103: output I/F
- 104: memory
- 105: storage
- 106: communication I/F
- 107: I/O port
- 108: internal bus
- 110: walking mode analysis system
- 111: sensor
- 120: walking mode display terminal
- 121: CPU
- 122: memory
- 123: communication I/F
- 124: input I/F
- 125: output I/F
- 126: internal bus
- 130: network
- 210: operator program
- 220: data visualization program
- 230: skeleton data extraction program
- 240: feature amount calculation program
- 250: data analysis program

## Claims

1. A walking mode display method that displays a walking mode of a walker, comprising:
previously storing, for each measurement about walking of a plurality of measurement subjects, skeleton data showing a trajectory of a measurement point of the measurement subject in a three-dimensional space, pitch data defining one walking of the measurement subject in the skeleton data, and feature amount data calculated based on the skeleton data for one walking step of the measurement subject;
selecting measurement of a walker and a reference walker to be compared with the walker among the plurality of measurement subjects, the measurement being to be displayed on a display screen;
displaying a first walking model that displays, as an animation, walking for one walking step of the walker based on the skeleton data and the pitch data related to the measurement of the walker;
displaying a second walking model that displays, as an animation, walking for one walking step of the reference walker based on the skeleton data and the pitch data related to the measurement of the reference walker; and
displaying a magnitude of predetermined feature amount data related to the measurement of the walker and a magnitude of predetermined feature amount data related to the measurement of the reference walker in a comparable manner.

2. The walking mode display method according to claim 1, wherein
the predetermined feature amount data to be displayed on the display screen is selected from feature amount data having a large ratio of difference between the magnitude of the feature amount data related to the measurement of the walker and the magnitude of the feature amount data related to the measurement of the reference walker.

3. The walking mode display method according to claim 1, further comprising:
previously storing feature amount explanation data that explains a content of the feature amount data calculated based on the skeleton data for one walking step of the measurement subject; and
when any of the predetermined feature amount data displayed on the display screen is selected, displaying explanation of the selected feature amount data by the feature amount explanation data, and displaying the measurement point used for calculation of the selected feature amount data on the first walking model and the second walking model.

4. The walking mode display method according to claim 3, further comprising:
previously storing domain knowledge data, that is specialized knowledge related to the feature amount data calculated based on the skeleton data for one walking step of the measurement subject; and
displaying the specialized knowledge on the display screen when the domain knowledge data includes the specialized knowledge related to the selected feature amount data.

5. The walking mode display method according to claim 1, further comprising:
previously storing a walking mode analysis model configured to calculate, based on the feature amount data, a walking mode degree indicating a degree corresponding to a predetermined walking mode;
applying the feature amount data related to the measurement of the walker to the walking mode analysis model so as to calculate a first walking mode degree related to the measurement of the walker;
applying the feature amount data related to the measurement of the reference walker to the walking mode analysis model so as to calculate a second walking mode degree related to the measurement of the reference walker; and
displaying the first walking mode degree and the second walking mode degree on the display screen.

6. The walking mode display method according to claim 5, further comprising:
displaying, as the first walking mode degree, a walking mode degree having a largest value among those calculated by applying the feature amount data related to the measurement of the walker to a plurality of walking mode analysis models.

7. The walking mode display method according to claim 6, wherein
the predetermined feature amount data to be displayed on the display screen is selected from feature amount data having a large contribution in the walking mode analysis model related to the calculation of the first walking mode degree.

8. The walking mode display method according to claim 5, wherein
the walking mode analysis model is a supervised learning model created or updated using the feature amount data related to the measurement of the plurality of measurement subjects as learning data.

9. A walking mode display system comprising:
a walking mode analyzer; and
a walking mode display terminal connected to the walking mode analyzer via a network, wherein
the walking mode analyzer includes a first processor, a first memory, a storage, and a data analysis program to be read into the first memory and executed by the first processor,
the walking mode display terminal includes a second processor, a second memory, and a data visualization program to be read into the second memory and executed by the second processor,
the storage of the walking mode analyzer previously stores, for each measurement about walking of a plurality of measurement subjects, skeleton data showing a trajectory of a measurement point of the measurement subject in a three-dimensional space, pitch data defining one walking of the measurement subject in the skeleton data, and feature amount data calculated based on the skeleton data for one walking step of the measurement subject,
the data visualization program of the walking mode display terminal includes:
an input and output processing unit configured to receive selection of measurement of a walker and a reference walker to be compared with the walker among the plurality of measurement subjects, the measurement being to be displayed on a display screen;
a skeleton data display processing unit configured to display a walking model that displays, as an animation, a walking for one walking step of each of the walker and the reference walker; and
a feature amount data display processing unit configured to display a magnitude of predetermined feature amount data related to the measurement of the walker and a magnitude of predetermined feature amount data related to the measurement of the reference walker in a comparable manner, and
the data analysis program of the walking mode analyzer includes:
a skeleton data acquisition processing unit configured to extract the skeleton data for one walking step related to the measurement of the walker and the measurement of the reference walker from the storage and transmit the skeleton data to the skeleton data display processing unit; and
a feature amount data acquisition processing unit configured to extract the predetermined feature amount data related to the measurement of the walker and the measurement of the reference walker from the storage and transmit the extracted feature amount data to the feature amount data display processing unit.

10. The walking mode display system according to claim 9, wherein
the feature amount data acquisition processing unit selects the predetermined feature amount data from feature amount data having a large ratio of difference between a magnitude of the feature amount data related to the measurement of the walker and a magnitude of the feature amount data related to the measurement of the reference walker.

11. The walking mode display system according to claim 9, wherein
the storage of the walking mode analyzer previously stores a walking mode analysis model configured to calculate, based on the feature amount data, a walking mode degree indicating a degree corresponding to a predetermined walking mode,
the data visualization program of the walking mode display terminal includes a walking mode degree display processing unit configured to display walking mode degrees respectively related to the measurement of the walker and the measurement of the reference walker, and
the data analysis program of the walking mode analyzer includes a walking mode quantification processing unit configured to apply the feature amount data, that is related to the measurement of the walker and the measurement of the reference walker and is extracted from the storage, to the walking mode analysis model, so as to calculate the walking mode degrees related to the measurement of the walker and the measurement of the reference walker, and transmit the walking mode degrees to the walking mode degree display processing unit.

12. The walking mode display system according to claim 11, wherein
the walking mode quantification processing unit transmits a walking mode degree having a largest value among those calculated by applying the feature amount data related to the measurement of the walker to a plurality of walking mode analysis models, to the walking mode degree display processing unit as the walking mode degree related to the measurement of the walker.

13. A walking mode analyzer connected to a walking mode display terminal via a network, comprising:
a processor;
a memory;
a storage; and
a data analysis program to be read into the memory and executed by the processor, wherein
the storage previously stores, for each measurement about walking of a plurality of measurement subjects, skeleton data showing a trajectory of a measurement point of the measurement subject in a three-dimensional space, pitch data defining one walking of the measurement subject in the skeleton data, and feature amount data calculated based on the skeleton data for one walking step of the measurement subject, and
the data analysis program includes:
a skeleton data acquisition processing unit configured to extract the skeleton data for one walking step related to measurement of a walker and measurement of a reference walker from the storage in order to display the walking for one walking step of each of the walker and the reference walker to be compared to the walker as an animation on the walking mode display terminal; and
a feature amount data acquisition processing unit configured to extract predetermined feature amount data related to the measurement of the walker and the measurement of the reference walker from the storage in order to display a magnitude of the predetermined feature amount data related to the measurement of the walker and a magnitude of the predetermined feature amount data related to the measurement of the reference walker on the walking mode display terminal in a comparable manner.

14. The walking mode analyzer according to claim 13, wherein
the feature amount data acquisition processing unit selects the predetermined feature amount data from feature amount data having a large ratio of difference between the magnitude of the feature amount data related to the measurement of the walker and the magnitude of the feature amount data related to the measurement of the reference walker.

15. The walking mode analyzer according to claim 13, wherein
the storage previously stores a walking mode analysis model configured to calculate, based on the feature amount data, a walking mode degree indicating a degree corresponding to a predetermined walking mode, and
the data analysis program includes a walking mode quantification processing unit configured to apply the feature amount data, that is related to the measurement of the walker and the measurement of the reference walker and is extracted from the storage, to the walking mode analysis model so as to calculate walking mode degrees related to the measurement of the walker and the measurement of the reference walker, in order to display the walking mode degrees related to the measurement of the walker and the measurement of the reference walker on the walking mode display terminal.

16. The walking mode analyzer according to claim 15, wherein
the walking mode quantification processing unit sets a walking mode degree having a largest value among those calculated by applying the feature amount data related to the measurement of the walker to a plurality of walking mode analysis models, as the walking mode degree related to the measurement of the walker.
